(19) Europäisches Patentamt European Patent Office Office européen des brevets

(11) **EP 4 306 047 A1**

(12) **EUROPEAN PATENT APPLICATION**

(43) Date of publication:
**17.01.2024 Bulletin 2024/03**

(21) Application number: **22184481.4**

(22) Date of filing: **12.07.2022**

(51) International Patent Classification (IPC):
***A61B 5/11*** (2006.01)     ***A61B 5/16*** (2006.01)
***A61B 5/00*** (2006.01)

(52) Cooperative Patent Classification (CPC):
**A61B 5/1101; A61B 5/1114; A61B 5/16;
A61B 5/4082; A61B 5/4088**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **KLAMING, Laura
Eindhoven (NL)**
• **HUIJBERS, Willem
Eindhoven (NL)**
• **VAN ELSWIJK, Gijs Antonius Franciscus
5656AG Eindhoven (NL)**
• **VAN EE, Raymond
5656AG Eindhoven (NL)**
• **VAN DOOREN, Marieke
Eindhoven (NL)**
• **SPELT, Hanne Adriana Alijda
Eindhoven (NL)**

(74) Representative: **Philips Intellectual Property &
Standards
High Tech Campus 52
5656 AG Eindhoven (NL)**

(54) **COGNITIVE TEST WORKFLOW ADJUSTMENT**

(57)    A method for adjustment of a cognitive testing routine in dependence upon detection of a motor impairment of the patient during performance of the cognitive testing routine. In particular, a workflow for administering a neuropsychological assessment which comprises one or more cognitive tests is adjusted in dependence upon detection of a motor impairment.

FIG. 2

EP 4 306 047 A1

**Description**

FIELD OF THE INVENTION

The present invention relates to the field of cognitive testing, and in particular to the configuration of a workflow of a cognitive test battery.

BACKGROUND OF THE INVENTION

Many older people may develop motor impairments such as tremor in the hands, bradykinesia, or speech tremors. There is a high prevalence of motor impairments in Parkinson's disease (PD), Essential Tremor (ET), and Multiple Sclerosis. Sometimes, motor impairments are a symptom of an underlying neurodegenerative disease (e.g. PD), but in other cases motor impairments can be benign, and might be driven by medication or other causes. Since patients with cognitive impairment and/or neurodegenerative diseases are the main target group of neuropsychological assessments (e.g. on the Philips IntelliSpace Cognition (ISC) platform), a considerable number of the subjects of neuropsychological assessments will have some form of motor impairment. Especially in early stages, motor impairments can be subtle, remain unreported by patients, and thus unknown to healthcare providers.

The most common motor impairment is tremor. This is an involuntary, rhythmic muscle contraction that leads to shaking movements in one or more parts of the body. Tremor often affects the hands, but can also affect the arms, head, vocal cords, torso, and legs. Tremor can be intermittent or constant. Tremor that affects the hands or arms can result in difficulty holding a pen and in writing or drawing. Vocal tremor can lead to a shaky voice that may be harder to understand.

SUMMARY OF THE INVENTION

Performance on cognitive tests that include a motor component can be impaired due to motor impairments, e.g. because it may take a patient longer to complete a test or because a patient may make more mistakes during a test. All tests on the ISC Platform for example have a motor component and can therefore be affected by motor impairments. Tests that involve drawing or the use of a pen and can be affected by tremor in the hands or bradykinesia. These include for example the Clock Drawing Test (CDT), Trail Making Test (TMT), and the Rey Osterrieth Complex Figure Test (ROCFT). Tests that involve speaking and can be affected by speech tremors include e.g. the Rey Auditory Verbal Learning Test (RAVLT) and the Controlled Oral Word Association Test (COWAT). Motor impairments can decrease performance on these tests due to the motor impairment rather than cognitive impairment.

Thus, it has been recognized by the inventors that there would be benefit in a system and method which can assist in automatically determining whether a particular result on a cognitive test has been affected by a motor impairment.

The invention is defined by the claims.

An aspect of the invention is a computer-implemented method comprising:

controlling a test apparatus to administer a neuropsychological assessment to a subject which includes a motor component, wherein the neuropsychological assessment includes administering a plurality of cognitive tests in accordance with an ordered schedule defined by a neuropsychological assessment workflow and processing individual test results according to a processing operation defined by the neuropsychological assessment workflow; automatically detecting one or more motor impairments during performance of the neuropsychological assessment; and

generating a neuropsychological assessment workflow change recommendation responsive to said detection; and generating an output indicative of the workflow change recommendation.

An aim according to the embodiments of the present invention is to provide a tool which can aid in the differential detection of motor impairments as a symptom of neurodegenerative disease, as distinct from cognitive impairment. In the proposed method, this is based on detecting motor impairment during the performing of a cognitive test and adjusting the testing workflow to compensate. This adjustment could be in the way in which the cognitive test scores are computed, and/or could be in the way that the test is conducted.

The neuropsychological assessment may include a battery of cognitive tests, e.g. the IntelliSpace Cognition (ISC) cognitive test battery.

In some embodiments, the method may further comprise implementing the workflow change recommendation by changing the workflow in accordance with the recommendation.

In some embodiments, the processing operation comprises combining scores obtained for a plurality of individual cognitive tests of the neuropsychological assessment into one or more cognitive domain scores according to a pre-associated cognitive domain category of each cognitive test.

In some embodiments, the cognitive domain scores include scores for one or more of: memory, working memory, processing speed, verbal processing, executive functioning, visual spatial processing.

The workflow adjustment recommendation may comprises recommendation of a correction of one or more cognitive domain scores.

This is to ensure that individual test scores as well as domain test scores are not unduly affected by motor impairments.

In some embodiments, the processing operation comprises combining a plurality of cognitive domain scores into an overall cognition score, and wherein the workflow adjustment recommendation comprises a recommendation of correction of said overall cognition score.

The workflow adjustment can be effected at different time points relative to the test being carried out.

In some embodiments, responsive to detecting a motor impairment during administering of a particular cognitive test, a user-perceptible output indicative of the workflow adjustment recommendation is generated during the neuropsychological assessment, and wherein the workflow change recommendation includes to stop the current cognitive test for which a motor impairment has been detected.

In some embodiments, responsive to detecting a motor impairment during the neuropsychological assessment, a user-perceptible output indicative of the recommendation is generated during the assessment, and wherein the workflow change recommendation includes to skip subsequent tests scheduled according to the workflow that involve a same motor component as the cognitive test for which the motor impairment has been detected.

In some embodiments, responsive to detecting a motor impairment during the neuropsychological assessment, an output indicative of the recommendation is generated after all tests scheduled according to the workflow have been administered, and wherein the workflow change recommendation includes that a score combination part of the processing operation is adjusted to compensate for scores obtained for any individual cognitive tests for which the motor impairment have been detected.

In some embodiments, scores for any tests for which motor impairment has been detected are removed from a score combination computation.

In some embodiments, the workflow change recommendation may only be generated only responsive to a determined degree of severity of a detected motor impairment exceeding a pre-defined threshold and not generated otherwise.

In some embodiments, the workflow further includes the generating of a clinical report indicative of results of the neuropsychological assessment and wherein the workflow change recommendation includes the adding of an indication of any detected motor impairment to the report.

In some embodiments, the detecting of motor impairments includes detecting presence of any one or more of: motor tremor in extremities, speech tremor, bradykinesia. These examples are of course non-exhaustive.

In some embodiments, the method further comprises determining a quantification of a severity of a detected motor impairment.

In some embodiments, administering of at least one of the cognitive tests includes the acquisition of a test dataset which includes one or more data variables associated with performance of the test.

There are different ways of detecting the one or more motor impairments.

One approach is through spectral analysis.

In this regard, in some embodiments, the detecting the one or more motor impairments comprises processing of at least a subset of the test dataset with a spectral analysis to derive a spectral power spectrum, and wherein the detecting of at least one motor impairment is performed based on analysis of the spectral power spectrum. The spectral analysis may comprise a Fourier analysis, and wherein the spectral power spectrum is a Fourier power spectrum. By way of further example, the spectral analysis may comprise a Wavelet analysis.

In some embodiments, a motor impairment to be detected may include a motor tremor. The aforementioned one or more data variables may include a data variable indicative of a motion of a part of the subject's body as a function of time.

In some embodiments, a motor impairment to be detected may include a voice tremor. The aforementioned one or more data variables may include a data variable indicative of voice acoustic data of a spoken input of the subject as a function of time.

In some embodiments, the motor impairment is determined based on detecting deviations between the derived spectral power spectrum for the subject and a pre-stored normal spectral power spectrum for a healthy control group.

In some embodiments, a degree of severity of the tremor is quantified based on the derived spectral power spectrum.

In some embodiments, where the administering of at least one of the cognitive tests includes the acquisition of a test dataset which includes a plurality of data variables associated with performance of the test, the detecting the one or more motor impairments comprises application of a Bayesian model to the plurality of data variables collected for the test, wherein the Bayesian model is configured to output a probability score indicative of a likelihood of presence of a pre-determined motor impairment. This provides a multimodal detection of motor impairment.

In some embodiments, one or a plurality of Bayesian models are applied to generate respective probability scores for a plurality of different motor impairments, and wherein the plurality of probability scores are combined into a combined probability score, and wherein the workflow change recommendation is generated only responsive to the combined probability score exceeding a pre-defined threshold.

The combining may comprise forming a weighted combination.

Weighting factors can optionally automatically be estimated based on variability in past and current performance.

In some embodiments, the detecting the motor impairment comprises differentially identifying a type of motor impairment which has been detected, and wherein the workflow change recommendation is configured in dependence upon the type(s) of cognitive impairment detected.

In some embodiments, the method further comprises a step of determining a risk prediction of cognitive function decline based on the neuropsychological assessment.

In some embodiments, the administering of at least one of the cognitive tests includes the acquisition of a test dataset which includes one or more data variables associated with performance of the test, and the method further comprises a calibration phase comprising acquiring a comparator dataset comprising the same data variables collected during said at least one cognitive test, but without administering said at least one cognitive test.

In some embodiments, the comparator dataset is analyzed to detect presence of, and to quantify a degree of severity of, a same motor impairment as has been detected during the administering of the cognitive test.

The inventive concepts can also be embodied in software form.

Another aspect of this invention is thus a computer program product comprising code means configured, when executed by a processor, to cause the processor to perform a method in accordance with any example or embodiment outlined in this document, or in accordance with any claim of this application.

The invention can also be embodied in hardware form.

Accordingly, another aspect of the invention is a system.

The system may comprise: an input/output, and one or more processors adapted to:

control, via control signals communicated though the input/output, a test apparatus to administer a neuropsychological assessment to a subject which includes a motor component, wherein the neuropsychological assessment includes administering a plurality of cognitive tests in accordance with an ordered schedule defined by a neuropsychological assessment workflow and processing individual test results according to a processing operation defined by the neuropsychological assessment workflow;

automatically detecting one or more motor impairments during performance of the neuropsychological assessment;

generating a neuropsychological assessment workflow change recommendation responsive to said detection; and

generating an output indicative of the workflow change recommendation.

The system may further include a memory storing a record of a standard neuropsychological workflow, the standard neuropsychological workflow defining an ordered schedule of cognitive tests to be administered to a subject, and defining a processing operation for processing individual test results.

The system may further comprise the test apparatus for administering the neuropsychological assessment to a subject.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a contribution of different cognitive tests to different cognitive domains;

Fig. 2 outlines steps of a method in accordance with one or more embodiments;

Fig. 3 is a block diagram of components of an example system in accordance with one or more embodiments of the invention;

Fig. 4 schematically illustrates a process of analyzing a cognitive test dataset to detect presence and/or severity of a motor impairment in accordance with a first example;

Fig. 5 schematically illustrates a process of analyzing a cognitive test dataset to detect presence and/or severity of a motor impairment in accordance with a second example; and

Fig. 6 schematically outlines a flow of steps of an example multimodal method for detection and analysis of a plurality of motor impairments and generating of a composite impairment score based thereon.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for adjustment of a cognitive testing routine in dependence upon detection of a motor impairment of the patient during performance of the cognitive test. In particular, a workflow for administering a neuropsychological assessment which comprises one or more cognitive tests is adjusted in dependence upon detection of a motor impairment.

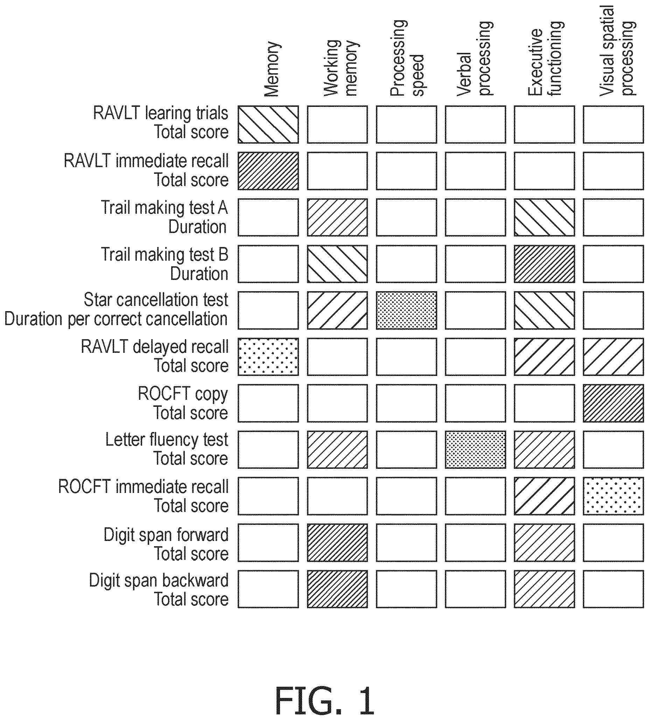

Digital neuropsychological assessment platforms include different cognitive tests. For instance, the ISC platform provides a battery of neuropsychological tests that assess different cognitive functions. Scores on individual neuropsychological tests are typically combined into cognitive domain scores according to a pre-associated cognitive domain category of each cognitive test. These might include for instance memory, verbal processing, executive functioning, and/or visual spatial processing. To illustrate, Fig. 1 shows an overview of cognitive tests available through the already mentioned Philips Intellispace Cognition (ISC) platform, and the contribution of each test to each of a set of exemplary cognitive domains. In the figure, the darkness of the grey indicates the magnitude of contribution to the relevant cognitive domain score, with the lightest grey indicating no contribution, and darker greys indicating smaller or larger contributions of a particular test to the cognitive domain in proportion to the darkness of the grey. Fig. 1 is an illustration from the ISC product manual.

The cognitive domain scores may then be amalgamated or combined into an overall cognition score in some cases.

The reader is also referred to the document: Pinter, N.K., Bottoms, A., Mann, C., Ajtai, B., Pasternak, J., Harlock, S., & Fritz, J.V. (n.d.). Digital cognitive assessment in clinical practice utilizing Philips IntelliSpace Cognition: early clinical and operational experience from the Dent Neurologic Institute, which provides a detailed discussion of the ISC tests and their application in clinical practice.

It can be seen from this that performance on an individual cognitive test therefore can impact the cognitive domain score and the overall cognition score, in embodiments in which such composite scores are calculated.

Thus, if the results for one or more cognitive tests are in fact invalid for a specific patient due to presence of a motor impairment which skews results, e.g. tremor, the scores on these cognitive tests would unduly affect the cognitive domain score and overall cognition score, and lead therefore to an inaccurate assessment of cognitive functioning. This may be of particular concern in cases where a neuropsychological assessment is a digital assessment and is performed in a remote or unsupervised manner. It is therefore important to know whether a motor impairment such as tremor has affected cognitive test performance. According to embodiments of this invention, this information can then be used to take remedial action by adjusting a workflow of the neuropsychological assessment to accommodate the motor impairment, e.g. adjusting individual cognitive test scores, discounting scores, removing specific tests from the domain and/or overall cognition scores, ceasing to perform a test or a variety of other workflow changes.

In accordance with this application, the inventors propose a method that automatically detects motor impairment during performance of a digital cognitive test battery (e.g. on the ISC platform already mentions) and makes a workflow recommendation. If a motor impairment is detected, a workflow change recommendation is made. As will become clear through the examples and discussion presented below, a workflow change recommendation may be understood as a change in the original or standard way of administrating tests on the particular digital neuropsychological assessment (e.g. the ISC platform) and of processing the individual test results and scores.

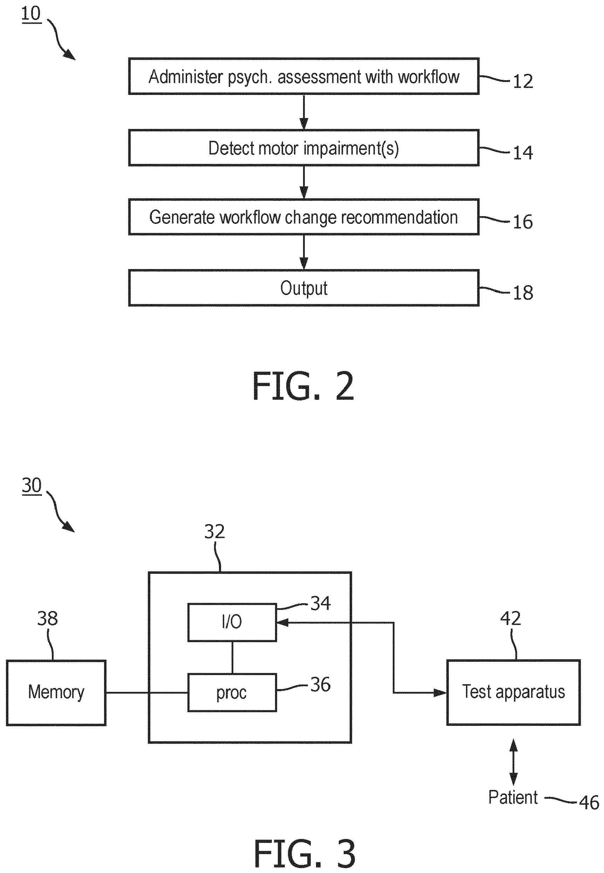

Fig. 2 outlines in block diagram form steps of an example method according to one or more embodiments. The steps will be recited in summary, before being explained further in the form of example embodiments.

The method 10 comprises controlling a test apparatus to administer 12 a neuropsychological assessment to a subject which includes a motor component.
The neuropsychological assessment includes administering a plurality of cognitive tests in accordance with an ordered schedule defined by a neuropsychological assessment workflow. The neuropsychological assessment may further include processing individual cognitive test results according to a processing operation defined by the neuropsychological assessment workflow.

The method 10 further comprises automatically detecting 14 one or more motor impairments during performance of the neuropsychological assessment. By way of illustration, motor impairments that could be detected might include any one or more of: motor tremor in extremities, speech tremor, and bradykinesia.

The method 10 further comprises generating 16 a neuropsychological assessment workflow change recommendation responsive to said detection.

The method 10 further comprises generating 18 an output indicative of the workflow change recommendation.

The output 18 could be a user-perceptible output, so that the end result of the method is simply a recommendation communicated to a user, e.g. a patient taking the test, or a clinician overseeing the administration of the test.

The output could be a data output. The data output may be communicated to one or more further locations or addresses.

The method may further comprise implementing the workflow change recommendation by changing the workflow in accordance with the recommendation.

As noted above, the method can also be embodied in hardware form.

To illustrate, Fig. 3 presents a schematic representation of an example system 30 in accordance with one or more embodiments of the invention.

The illustrated system comprises a processing unit 32 comprising an input/output 34 and one or more processors 36.

The one or more processors 36 are configured to perform a method in accordance with any example or embodiment described in this document, or in accordance with any claim of this application. More explicitly, the one or more processors 36 may be adapted to perform the following steps:

control, via control signals communicated though the input/output 34, a test apparatus 42 to administer a neuropsychological assessment to a subject which includes a motor component, and wherein the neuropsychological assessment includes administering a plurality of cognitive tests in accordance with an ordered schedule defined by a neuropsychological assessment workflow and processing individual test results according to a processing operation defined by the neuropsychological assessment workflow;

automatically detect one or more motor impairments during performance of the neuropsychological assessment;

generate a neuropsychological assessment workflow change recommendation responsive to said detection; and

generate an output indicative of the workflow change recommendation.

An aspect of the invention is a system comprising just the processing unit 32 (or expressed another way, just the input/output 34 and the one or more processors 36).

Optionally, the system 30 can further comprise a memory 38 storing a record of a standard neuropsychological workflow, the standard neuropsychological workflow defining an ordered schedule of cognitive tests to be administered to a subject, and defining a processing operation for processing individual test results.

Optionally, the system may comprise a test apparatus 42 for administering a neuropsychological assessment to a subject. For example, this may be a computer-based testing apparatus, i.e. comprising a computing device. It may be a dedicated computing console or terminal. It may comprise a mobile computing device such as a tablet computer, smartphone or laptop. It may include a user interface. The user interface may include one or more sensory output devices for generating user-perceptible stimuli. The user interface may include a user input apparatus. This may include one or more pointer devices, such as an electronic pen or pencil, and/or a touchscreen, and/or a gesture capture device such as a handheld unit with an inertial measurement unit integrated therein or a camera/sensor based gesture capture device.

In any embodiment, the neuropsychological assessment may be a digital neuropsychological assessment. A digital neuropsychological assessment comprises a neuropsychological assessment administered through control of a user interface device to generate prompt instructions and measure test response data, i.e. an electronically administered test.

As noted above, in a typical neuropsychological assessment workflow, scores for individual neuropsychological tests are combined into cognitive domain scores, e.g. memory, executive functioning. Cognitive domain scores may be further amalgamated or combined into an overall cognition score.

Thus, in some embodiments of the invention, the processing operation part of the workflow comprises combining scores obtained for a plurality of individual cognitive tests of the neuropsychological assessment into one or more cognitive domain scores according to a pre-associated cognitive domain category of each cognitive test. This combining operation may, in the standard workflow, be defined by a combining function which specifies a weighting to be attached to each individual cognitive test score in the combination.

By way of example, the cognitive domain scores might include any scores for any one or more of: memory, verbal processing, executive functioning, visual spatial processing.

The workflow adjustment recommendation may in some embodiments comprise recommendation of a correction of one or more cognitive domain scores. It might additionally or alternatively comprise a change to the weighting attached to one or more individual cognitive test scores in the combination forming the cognitive domain score. It might additionally or alternatively include exclusion of one or more cognitive test scores from the cognitive domain score calculation.

Furthermore, in accordance with some embodiments, the processing operation part of the workflow may comprise combining a plurality of cognitive domain scores into an overall cognition score. Here, in some embodiments, the workflow adjustment recommendation comprises a recommendation of correction of said overall cognition score.

There are different options as to when, within the workflow of the neuropsychological assessment, the workflow change recommendation is made and/or implemented.

In some embodiments, the recommendation can be made during the neuropsychological assessment so that for example the test on which motor impairment has been detected is stopped.

For example, in some embodiments, responsive to detecting a motor impairment during administering of a particular cognitive test, a user-perceptible output indicative of the workflow adjustment recommendation is generated during the neuropsychological assessment, and wherein the workflow change recommendation includes to stop the current cognitive test for which a motor impairment has been detected.

By way of another example, the workflow change recommendation could be made during the assessment so that for example subsequent tests that involve the same motor component (i.e. drawing, writing, or speech) are skipped from the neuropsychological assessment.

For example, in some embodiments, responsive to detecting a motor impairment during the neuropsychological assessment, a user-perceptible output indicative of the recommendation is generated during the assessment, and wherein the workflow change recommendation includes to skip subsequent tests scheduled according to the workflow that involve a same motor component as the cognitive test for which the motor impairment has been detected.

In accordance with either of the above examples, the user-perceptible output might be a visual or auditory output for example.

In some embodiments, the workflow change recommendation may be made after the assessment so that tests for which a motor impairment has been detected are removed from the overall from a computation of relevant cognitive domain test scores and/or an overall cognition score.

For example, in some embodiments, responsive to detecting a motor impairment during the neuropsychological assessment, an output indicative of the recommendation is generated after all tests scheduled according to the workflow have been administered, and wherein the workflow change recommendation includes that a score combination part of the processing operation is adjusted to compensate for scores obtained for any individual cognitive tests for which the motor impairment have been detected. For example, scores for any tests for which motor impairment has been detected might be removed from a score combination computation.

In any of the above, it is an option that the workflow change recommendation is generated only responsive to a determined degree of severity of a detected motor impairment exceeding a pre-defined threshold and is not generated otherwise. Options will be discussed below as to how a degree of severity can be determined.

In some embodiments, the workflow may further include the generating of a clinical report indicative of results of the neuropsychological assessment.

In some embodiments, the workflow recommendation may include a warning or statement of a possible tremor added to the clinical report. This workflow change can shorten assessment times and ensures that the outcome of the overall cognitive assessment - or specific components - is reliable and valid.

With regards to the detecting of the motor impairment, different options are possible. In general, it is based on processing one or more data variables included in a test dataset acquired as part of the cognitive test with a dedicated detection algorithm which detects presence or absence or at least one particular motor impairment, and preferably determines a measure of its severity.

One advantageous approach is to use spectral analysis of the cognitive test dataset, e.g. Fourier analysis or Wavelet analysis.

In this approach, one or more motor impairments are detected based on processing of at least a subset of an acquired cognitive test dataset with a spectral analysis to derive a frequency domain power spectrum, for example a Fourier power spectrum, and wherein the detecting of at least one motor impairment is performed based on analysis of the spectral power spectrum. The cognitive test dataset is one which is acquired as part of the normal administration of the at least one of the cognitive tests, and it includes one or more data variables associated with performance of the test.

As a general principle, a given motor impairment can be detected and identified in this way based on detecting deviations between the derived spectral power spectrum for the subject and a pre-stored normal spectral power spectrum for a healthy control group. A dedicated comparison algorithm may be provided for differentially detecting each of one or more different motor impairments, based on applying different detection criteria to any detected deviations, and/or detecting or extracting the deviations in a different way.

In some embodiments, a degree of severity of a motor impairment can be quantified based on the derived spectral power spectrum.

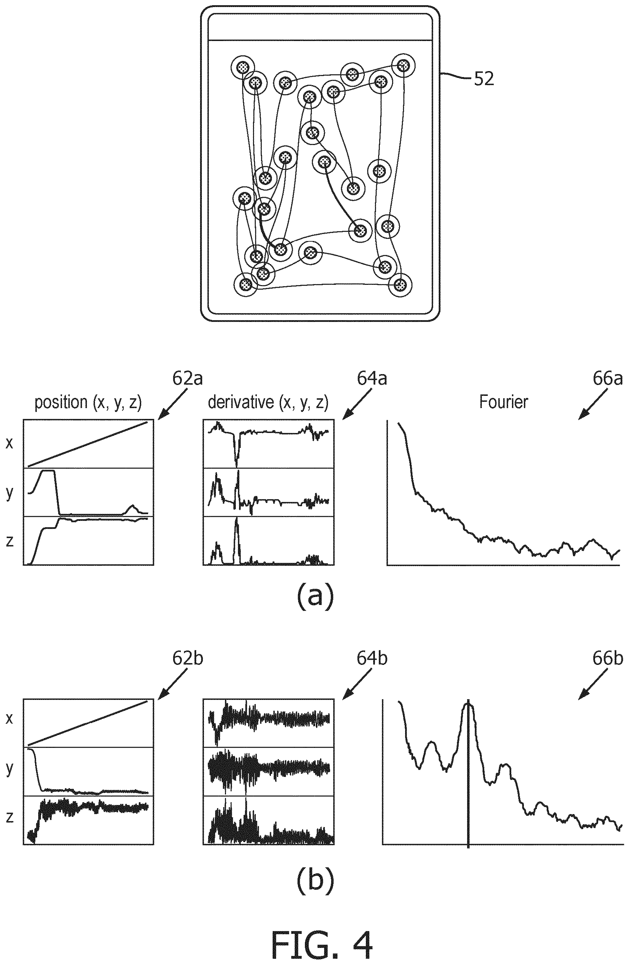

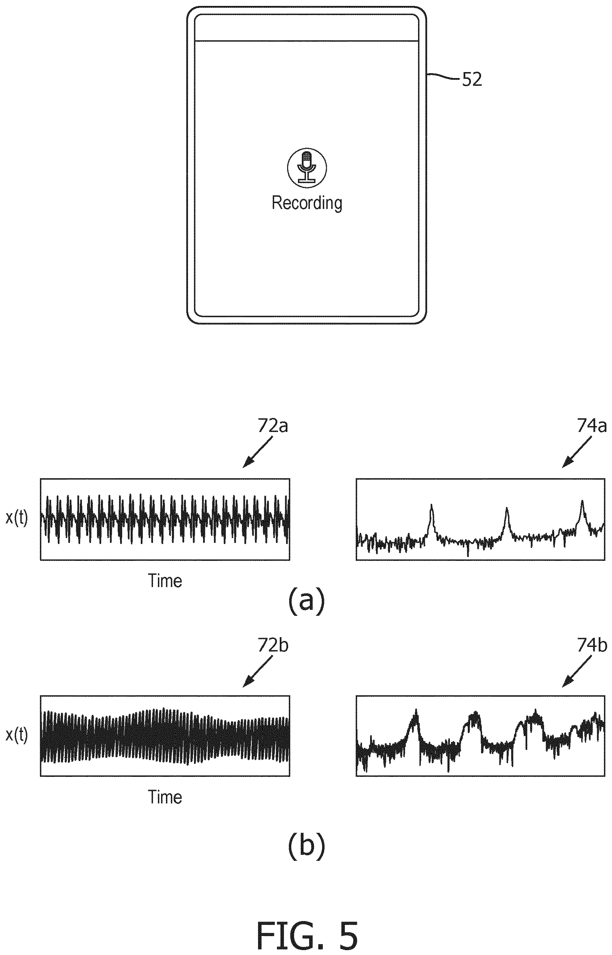

To illustrate this concept, two example implementations will now be described with reference to Fig. 4 and Fig. 5 respectively. It will be appreciated that not all features of each of these particular examples are essential to the inventive concept, and each is described to aid understanding and to provide an example to illustrate the inventive concepts.

A first example, which will be described with reference to Fig. 4, concerns a case in which the motor impairment to be detected includes a motor tremor. The cognitive test being administered is one which requires a user to perform a drawing task via movement of a pencil pointer device over an input means of a computing device. In the example of Fig. 4, the input device is a touchscreen display 52. The computing device is a tablet computer in the illustrated example.

A test dataset is acquired which includes a data variable corresponding to the motion path of the pencil pointer device over the screen 52.

Thus, this is an example of a more general case in which the administering of at least one of the cognitive tests of the battery includes the acquisition of a test dataset which includes a data variable which is indicative of a motion of a part of the subject's body as a function of time.

A quantification of a motor tremor is then determined according to this set of embodiments via automatic analysis of the tactile input, as provided via the pencil of the tablet. The analysis is schematically illustrated in Fig. 4. In this illustrated example, the test corresponds to the Trail Making Test (TMT) of the ISC platform mentioned above.

The process involves comparison of a Fourier spectrum derived from the captured tactile input (row b), with a baseline spectrum for a healthy population (row a)

The drawing trajectories captured by the input device 52 are processed to extract x, y and z position variables 62b as a function of time, i.e. x, y, z time series. This may involve a normalization process, using calibration data already acquired earlier in the workflow, for example via an instruction/test session or when the test subject is writing their name. This normalization could be done in real time while the input data is being acquired, i.e. while the user is drawing on the screen 52, or it could be done in batch form at a later stage.

The x, y and z position variable data are processed to obtain time x, y, and z first time derivative data 64b. A Fourier transform is then applied to the x, y, z first derivative signals 64b over a specified time window, and from this a Fourier power spectrum 66a is derived. As an alternative, the Fourier transform could be applied directly to the position variable signals 62a.

An analysis is performed comprising comparison or reference to a Fourier spectrum 66a for a healthy control. Fig. 4 schematically illustrates, to further aid understanding and explanation, the intermediate steps of deriving position trajectory data 62a, first derivative data 64a and the Fourier spectrum 66a for the healthy control population. However in practice, it is anticipated that these steps would be performed in advance of the method of the invention, with the Fourier spectrum pre-stored, ready for a comparison or reference with the newly captured data spectrum 66b to be made. However, it is of course also conceivable to perform the Fourier analysis of the control data in real time.

A deviation of the Fourier power spectrum 66b derived from the test dataset from the normal power spectrum 66a is determined, over an equivalent time window. From this, an estimate can be made of the frequency peaks in the test spectrum 66b corresponding to a tremor. The frequency band of each tremor, amplitudes of each tremor (e.g. from the height of each peak) and a number of tremors can be detected (e.g. from the number of peaks) can then be used to quantify the tremor.

In the illustrated example, a clear peak can be detected in the test Fourier spectrum 66b. The quantification (e.g. spectral power, or area-under-curve) of this peak, relative to the normal spectrum 66a, can be used to detect for example hand tremors.

A second example, which will be described with reference to Fig. 5, concerns a case in which the motor impairment to be detected includes a voice tremor. The detection and preferably quantification of the voice tremor is determined via automatic analysis of a speech input, as provided via a microphone, of for example a tablet computer 52.

Thus, this is an example of a more general case in which the administering of at least one of the cognitive tests of the battery includes the acquisition of a test dataset which includes a data variable which is indicative of voice acoustic data of a spoken input of the subject as a function of time. By way of example, voice-based cognitive tests in the ISC platform include the Rey Auditory Verbal Learning Test (RAVLT) and the Controlled Oral Word Association Test (COWAT).

Row (a) corresponds to data for a healthy control. Row (b) corresponds to data acquired for a test subject during a cognitive test who has a voice tremor. Acoustic amplitude data 72b is acquired during performance of the test, and to this spectral analysis is performed over a defined time window, in the form of wavelet analysis, to produce a power spectrum 74b in the frequency domain. Fourier analysis could instead be performed. Peaks in the power spectrum can then be derived. Based on deviation from the normal power spectrum 74a, derived from acoustic data 72a for a normal control population a motor impairment can be detected and quantified. For example, a number of detected deviations (e.g. number of peaks present in test data spectrum which are not present in the normal spectrum) can be used to quantify a voice tremor. Additionally or alternatively, an area under the curve of detected deviant peaks in the test spectrum 74b could be used to quantify a tremor, and/or a height of such peaks.

A more accurate or discriminative detection of one or more motor impairments may be possible if, instead of a single data variable, the administering of at least one of the cognitive tests includes the acquisition of a test dataset which includes a plurality of data variables associated with performance of the test and the plurality of data variables of the test dataset are included in the analysis. This is known in the art as multi-modal assessment.

For instance, with reference to the examples discussed above in Fig. 4 and Fig. 5, additionally, information about the velocity of pen movements and changes in velocity, the angle of the pen on the tablet and changes in the angle of the pen, the number and speed of direction changes, the pressure and changes in pressure during drawing and/or writing could be monitored and acquired as part of the test dataset. These data variables could then be used for multimodal detection of motor impairments that affect the hands and/or arms.

The multimodal assessment may be specific to the given cognitive test, and may take characteristics of the specific cognitive test into account. For instance, for the Trail Making Test (TMT), velocity changes are expected because of the visual distribution of targets. Velocity will decrease when a target is approached and will increase when a target is exited. Velocity will also be influenced by the distance between two consecutive targets. One solution would be to define a perimeter around targets and exclude velocity changes within this perimeter from analysis. Together, these indicators - velocity, angle, direction changes, pressure - can be used for multimodal detection of tremor.

Based on multiple measured test variables, or parameters derived therefrom such, such as from the spectral transform, a motor impairment "score" can be determined representative of a probability that a particular motor impairment, such as a tremor, occurred P(T|C), given both prior knowledge and the measured parameters of the test variables or spectral transform of the tremor during the task at hand.

A way of combining likelihoods and prior knowledge is according to Bayesian probability theory.

For example, as a general principle, where the administering of at least one of the cognitive tests includes the acquisition of a test dataset which includes a plurality of data variables, the detecting the one or more motor impairments in some embodiments may comprise application of a Bayesian model to the plurality of data variables, or parameters derived therefrom , for instance parameters of a spectral transform. The Bayesian model may, as mentioned above, be configured to output a probability score, P(T|C), indicative of a likelihood of presence of a pre-determined motor impairment.

Further details will now be elaborated by way of description of one example implementation in which the motor impairment to be detected is a tremor. However, it should be understood that the principles of the approach can be applied more broadly to detection of any motor impairment.

In Bayesian models, a generic inference is decomposed, first, into a 'likelihood' probability distribution. This represents the probability for one or more cues (C), given sensory motor impairment data (M), denoted by P(C|M). It is further decomposes, second, into a so-called 'prior'. The represents the *a priori* expected probability related to the sensory motor impairment data P(M). The likelihood and prior are combined to produce the 'posterior' (*a posteriori*) probability P(M|C), representing the probability that a particular parameter in sensory motor impairment data (M) is present given the cues (C), and is obtained from Bayes' rule with general form: $P(T|C) \propto P(C|T)P(T)$. If this score (probability) exceeds a predetermined threshold (e.g. a population average), a flag is raised in a log file. In addition, the (prior) conditions under which the threshold was exceeded are recorded in a log file.

Processes for building and implementing of suitable Bayesian models would be well known to a skilled person in this field. For example, reference is made to the Internet encyclopedia article: https://en.wikipedia.org/wiki/Bayes_theorem. Any textbook on Bayesian theory could also be used as further reference.

In some embodiments, a composite multimodal tremor "score" can be determined in order to quantify the role of multiple impairments (e.g. tremor in the hands, vocal tremor) combined.

In this regard, as a general principle, in some embodiments, one or a plurality of Bayesian models are applied to generate respective probability scores for a plurality of different motor impairments, and wherein the plurality of probability scores are combined into a combined probability score. In some embodiments, a quantitative value of this score can be used to determine whether a workflow change is needed, and also what workflow change to make. In some embodiments, the workflow change recommendation may only be generated responsive to the combined probability score exceeding a pre-defined threshold.

By way of an example, a patient could exhibit multiple tremors, each with a specific likelihood and prior conditions in which the tremor occurs. Combining the influence of multiple tremors would be valuable because multiple types of tremors can interfere with cognitive functioning in an accumulative way. Multiple individual tremor scores can be combined using weighting factors. The weighting factors can for example automatically be estimated based on variability in past and current performance (e.g. the variability in the above-mentioned parameters from the spectral power spectrum). For example, a composite tremor score could be calculated from scores for individual tremors as follows:

$$\text{Weight1 x Tremor1} + \text{Weight2 x Tremor2} = \text{Composite Tremor}$$

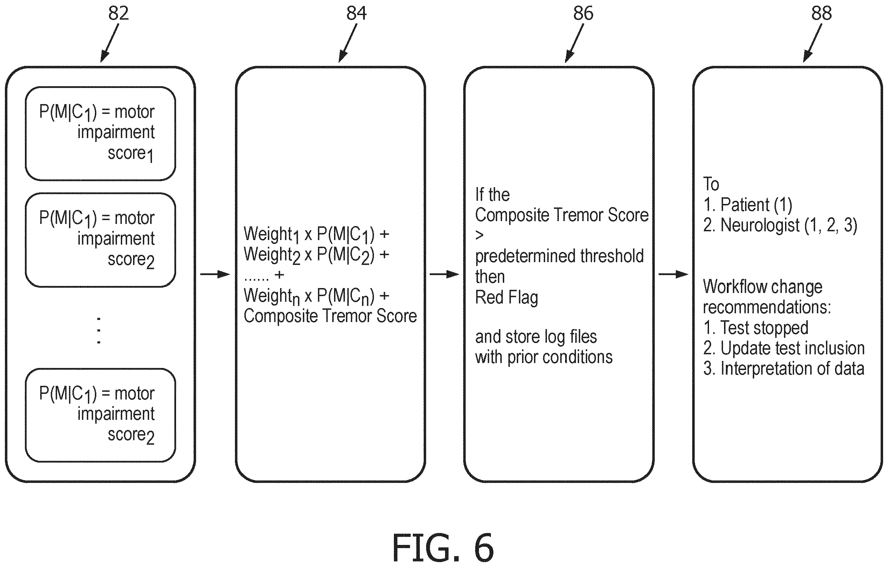

To summarize the concept, Fig. 6 outlines an example process flow for an exemplary Bayesian approach for motor impairment detection.

The process comprises, in a first stage 82, determining a respective motor impairment probability score for each of a plurality of different motor impairments, for example in a manner already discussed above.

The method further comprises, in a second stage 84, a multimodal processing operation in which the plurality of individual probability scores obtained in stage 82 are combined with respective weightings to form a composite motor impairment score (e.g. Composite Tremor score in the example of Fig. 6).

The method further comprises, in a third stage 86, a decision step in which the algorithm assesses the composite score with reference to one or more pre-defined criteria to determine presence of absence of a motor impairment. For example, in the illustrated case in Fig. 6, the composite score is compared against a pre-determined threshold.

The method further comprises, in stage 88, the generating of a recommendation based on the decision step of stage 86. This may be a user-perceptible recommendation and may be communicated to the patient, and/or to the neurologist administering the neuropsychological assessment. Different recommendation items, e.g. (1), (2) and (3) listed in the example of Fig. 6, may be communicated to different of the patient and the neurologist, e.g. a recommendation to stop completing the test is issued to the patient, and, in addition, recommendations to update the test result calculations, or interpretation communicated to the neurologist.

In some embodiments, different types of motor impairment can be distinguished from one another by means of the analysis applied in accordance with the invention, e.g. tremor can be distinguished from bradykinesia, tremor of the head is distinguished from a laryngeal (vocal) tremor. This would be valuable because some types of tremors are known to interfere more with cognitive functioning.

As a general principle therefore, it can be said that in some embodiments, the detecting the motor impairment comprises differentially identifying a type of motor impairment which has been detected, and wherein the workflow change recommendation is configured in dependence upon the type(s) of cognitive impairment detected.

The detection of a motor impairment can be used to inform further analysis or detection. For example, a risk of future cognitive impairment, or progression of cognitive impairment, could be predicted at least in part based on a detected motor impairment and severity or other parameters therefore (e.g. type). Thus, in some embodiments, the method may further comprise a step of determining a risk prediction of cognitive function decline based on the neuropsychological assessment.

For example, research has shown that tremor at the onset of Parkinson's Disease (PD) is a significant predictor of poorer cognitive performance. With regards to different types of tremor, research has shown that patients with PD with primary akinetic and rigidity type tremors, but not PD patients with primary tremor, have decreased activity in default mode networks and lower grey matter volume compared to healthy controls (Karunanayaka, P.R., Lee, E.-Y., Lewis, M.M., Sen, S., Eslinger, P.J., Yang, Q.X., & Huang, X. (2017). Default mode network differences between rigidity- and tremor-predominant Parkinson's disease. Cortex, 81, 239-250). Decreased activity in default mode networks is related to cognitive impairment in PD (*ibid*). Different variables, including magnitude and type of tremor, but also cognitive impairment (corrected for motor impairment) may for example be used to predict risk of (further) cognitive decline in the long-term in neurodegenerative diseases.

Besides a quantification of the severity of motor impairment during the administration of the cognitive test battery, there could be implemented a calibration phase in which parameters of the analysis are set so as to enable subsequent quantification of a potential motor impairment (e.g. according to detected data variables such as frequency, amplitude, intensity, asymmetry).

In particular, where the administering of at least one of the cognitive tests includes the acquisition of a test dataset which includes one or more data variables associated with performance of the test, in some embodiments, the method may further comprise a calibration phase comprising acquiring a comparator dataset comprising the same data variables collected during said at least one cognitive test, but without administering said at least one cognitive test.

For example, a dedicated calibration dataset of data variables can be acquired during rest, i.e. while no task is executed. Often, in e.g. PD, a tremor is worse in rest compared to when executing a planned movement. Once a movement plan is initiated and executed, the tremor can diminish in severity. Therefore, it would be interesting to compare the severity of tremor in rest (before test administration) with the severity of tremor during execution of the cognitive test battery.

During analysis of the cognitive test dataset to detect a motor impairment, the comparator dataset can be utilized to detect presence of, and to quantify a degree of severity of, a same motor impairment as has been detected during the administering of the cognitive test.

As already has been emphasized, although examples in this document have tended to focus on tremors, other types of motor impairment, e.g. bradykinesia or rigidity, can also be detected in a similar way and used for a workflow change recommendation in a similar way as described above. Bradykinesia (i.e. slowness of movement) and rigidity (i.e. stiffness and resistance to movement) are common symptoms in neurodegenerative diseases such as Parkinson's disease, and can also affect performance on cognitive tests in the absence of cognitive impairment. Similar as described above, detection of other types of motor impairment can be used to generate a workflow change recommendation (1) during the assessment so that the test on which motor impairment has been detected is stopped if the motor impairment exceeds a certain threshold, (2) during the assessment so that subsequent tests that involve the same motor component (i.e. drawing, writing, or speech) are skipped from the assessment if the motor impairment exceeds a certain threshold, and/or (3) after the assessment so that tests on which motor impairment has been detected are removed from the overall cognitive model as well as from individual and domain test scores if the motor impairment exceeded a certain threshold.

In another embodiment, disease-specific norm scores might be used to correct cognitive test scores in dependence upon a determined level of tremor. In that way, rather than removing entirely from computation of a composite score test scores for a test for which a tremor has been detected, it is still be possible to assess cognitive functioning in patient groups with tremor longitudinally.

Embodiments of the invention described above employ one or more processors. The one or more processors may be located in a single containing device, structure or unit, or may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The one or more processors can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to".

Any reference signs in the claims should not be construed as limiting the scope.

**Claims**

**1.** A computer-implemented method (10) comprising:

controlling (12) a test apparatus to administer a neuropsychological assessment to a subject which includes a motor component, wherein the neuropsychological assessment includes administering a plurality of cognitive tests in accordance with an ordered schedule defined by a neuropsychological assessment workflow and processing individual test results according to a processing operation defined by the neuropsychological assessment workflow;
automatically detecting (14) one or more motor impairments during performance of the neuropsychological assessment; and
generating (16) a neuropsychological assessment workflow change recommendation responsive to said detection;
generating (18) an output indicative of the workflow change recommendation.

**2.** The method of claim 1, further comprising implementing the workflow change recommendation by changing the workflow in accordance with the recommendation.

**3.** The method of claim 1 or 2, wherein the processing operation comprises combining scores obtained for a plurality of individual cognitive tests of the neuropsychological assessment into one or more cognitive domain scores according to a pre-associated cognitive domain category of each cognitive test, and optionally wherein the cognitive domain scores include scores for one or more of: memory, verbal processing, executive functioning, visual spatial processing, and

wherein the workflow adjustment recommendation comprises recommendation of a correction of one or more cognitive domain scores.

4. The method of any of claims 1-3, wherein, responsive to detecting a motor impairment during administering of a particular cognitive test, a user-perceptible output indicative of the workflow adjustment recommendation is generated during the neuropsychological assessment, and wherein the workflow change recommendation includes to stop the current cognitive test for which a motor impairment has been detected.

5. The method of any of claims 1-4, wherein, responsive to detecting a motor impairment during the neuropsychological assessment, a user-perceptible output indicative of the recommendation is generated during the assessment, and wherein the workflow change recommendation includes to skip subsequent tests scheduled according to the workflow that involve a same motor component as the cognitive test for which the motor impairment has been detected.

6. The method of any of claims 1-5, wherein, responsive to detecting a motor impairment during the neuropsychological assessment, an output indicative of the recommendation is generated after all tests scheduled according to the workflow have been administered, and wherein the workflow change recommendation includes that a score combination part of the processing operation is adjusted to compensate for scores obtained for any individual cognitive tests for which the motor impairment have been detected, and optionally wherein scores for any tests for which motor impairment has been detected are removed from a score combination computation.

7. The method of any of claims 1-6, wherein the method further comprises determining a quantification of a severity of a detected motor impairment.

8. The method of any of claims 1-7,

wherein the administering of at least one of the cognitive tests includes the acquisition of a test dataset which includes one or more data variables associated with performance of the test, and
wherein the detecting the one or more motor impairments comprises processing of at least a subset of the test dataset with a spectral analysis to derive a spectral power spectrum, and wherein the detecting of at least one motor impairment is performed based on analysis of the spectral power spectrum.

9. The method of claim 8, wherein a motor impairment to be detected includes a motor tremor, and wherein the one or more data variables include a data variable indicative of a motion of a part of the subject's body as a function of time.

10. The method of claim 8 or 9, wherein a motor impairment to be detected includes a voice tremor, and wherein the one or more data variables include a data variable indicative of voice acoustic data of a spoken input of the subject as a function of time.

11. The method of any of claims 8-10, wherein a degree of severity of the tremor is quantified based on the derived spectral power spectrum.

12. The method of any preceding claim,

wherein the administering of at least one of the cognitive tests includes the acquisition of a test dataset which includes a plurality of data variables associated with performance of the test, and
wherein the detecting the one or more motor impairments comprises application of a Bayesian model to the plurality of data variables collected for the test, wherein the Bayesian model is configured to output a probability score indicative of a likelihood of presence of a pre-determined motor impairment.

13. The method of claim 12, wherein one or a plurality of Bayesian models are applied to generate respective probability scores for a plurality of different motor impairments, and wherein the plurality of probability scores are combined into a combined probability score, and wherein the workflow change recommendation is generated only responsive to the combined probability score exceeding a pre-defined threshold.

14. A computer program product comprising code means configured, when executed by a processor, to cause the processor to perform a method in accordance with any of claims 1-13.

15. A system (30) comprising:

an input/output (34); and
one or more processors (36) adapted to:

control, via control signals communicated though the input/output, a test apparatus (42) to administer a neuropsychological assessment to a subject which includes a motor component wherein, the neuropsychological assessment includes administering a plurality of cognitive tests in accordance with an ordered schedule defined by a neuropsychological assessment workflow and processing individual test results according to a processing operation defined by the neuropsychological assessment workflow,
automatically detect one or more motor impairments during performance of the neuropsychological assessment,
generate a neuropsychological assessment workflow change recommendation responsive to said detection, and
generate an output indicative of the workflow change recommendation.

|  | Memory | Working memory | Processing speed | Verbal processing | Executive functioning | Visual spatial processing |
|---|---|---|---|---|---|---|
| RAVLT learing trials Total score | ▨ |  |  |  |  |  |
| RAVLT immediate recall Total score | ▨ |  |  |  |  |  |
| Trail making test A Duration |  | ▨ |  |  | ▨ |  |
| Trail making test B Duration |  | ▨ |  |  | ▨ |  |
| Star cancellation test Duration per correct cancellation |  | ▨ | ▨ |  | ▨ |  |
| RAVLT delayed recall Total score | ▨ |  |  |  | ▨ | ▨ |
| ROCFT copy Total score |  |  |  |  |  | ▨ |
| Letter fluency test Total score |  | ▨ |  | ▨ | ▨ |  |
| ROCFT immediate recall Total score |  |  |  |  | ▨ | ▨ |
| Digit span forward Total score |  | ▨ |  |  | ▨ |  |
| Digit span backward Total score |  | ▨ |  |  | ▨ |  |

# FIG. 1

10

| Administer psych. assessment with workflow | —12 |

↓

| Detect motor impairment(s) | —14 |

↓

| Generate workflow change recommendation | —16 |

↓

| Output | —18 |

# FIG. 2

30

32

| Memory | 38 |

| 32 |
| I/O | —34 |
| proc | —36 |

| Test apparatus | 42 |

Patient —46

# FIG. 3

FIG. 4

x(t) 72a

Time

74a

x(t) 72b

Time

74b

(a)

(b)

FIG. 5

82

P(M|C$_1$) = motor impairment score$_1$

P(M|C$_1$) = motor impairment score$_2$

. . .

P(M|C$_1$) = motor impairment score$_2$

84

Weight$_1$ x P(M|C$_1$) +
Weight$_2$ x P(M|C$_2$) +
...... +
Weight$_n$ x P(M|C$_n$) +
Composite Tremor Score

86

If the
Composite Tremor Score
>
predetermined threshold
then
Red Flag

and store log files
with prior conditions

88

To
1. Patient (1)
2. Neurologist (1, 2, 3)

Workflow change
recommendations:
1. Test stopped
2. Update test inclusion
3. Interpretation of data

FIG. 6

Europäisches
Patentamt

European
Patent Office

Office européen
des brevets

**EUROPEAN SEARCH REPORT**

Application Number

EP 22 18 4481

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/13687 A1 (BOWLES LANGLEY TECHNOLOGY INC [US]) 21 February 2002 (2002-02-21) * paragraphs [0004] - [0005], [0008] - [0010]; figure 1 * ----- | 1-4,7-15 | INV. A61B5/11 A61B5/16 A61B5/00 |
| X | US 2003/233032 A1 (TEICHER MARTIN H [US] ET AL) 18 December 2003 (2003-12-18) * paragraphs [0013] - [0014], [0023], [0034] - [0039]; figure 1 * ----- | 1-4,7,8, 11-15 | |
| X | COSTABILE TERESA ET AL: "Application of the p9NORM correction method to timed neuropsychological tests in Parkinson's disease and multiple system atrophy", NEUROLOGICAL SCIENCES (TESTO STAMPATO), SPRINGER VERLAG, MILAN, IT, vol. 41, no. 12, 28 May 2020 (2020-05-28), pages 3633-3641, XP037290129, ISSN: 1590-1874, DOI: 10.1007/S10072-020-04478-3 [retrieved on 2020-05-28] * page 3633 - page 3635; table 1 * * page 3639 * ----- | 1-4,6-8, 11-15 | TECHNICAL FIELDS SEARCHED (IPC) A61B |
| X | MONICA FALAUTANO: "Neuropsychological assessment: experimental and clinical research", NEUROLOGICAL SCIENCES ; OFFICIAL JOURNAL OF THE ITALIAN NEUROLOGICAL SOCIETY, SPRINGER-VERLAG, MI, vol. 31, no. 2, 17 July 2010 (2010-07-17), pages 223-226, XP019865579, ISSN: 1590-3478, DOI: 10.1007/S10072-010-0371-9 * page 244 * ----- -/-- | 1-5,7,8, 11-15 | |

The present search report has been drawn up for all claims

| Place of search | Date of completion of the search | Examiner |
|---|---|---|
| Berlin | 8 December 2022 | Dydenko, Igor |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 2

Europäisches
Patentamt

European
Patent Office

Office européen
des brevets

# EUROPEAN SEARCH REPORT

Application Number

**EP 22 18 4481**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JAEGER JUDITH ET AL: "Development of a cognitive composite for measuring change in progressive supranuclear palsy", PARKINSONISM AND RELATED DISORDERS, ELSEVIER SCIENCE, OXFORD, GB, vol. 92, 12 October 2021 (2021-10-12), pages 94-100, XP086882479, ISSN: 1353-8020, DOI: 10.1016/J.PARKRELDIS.2021.10.007 [retrieved on 2021-10-12] * the whole document * | 1-15 | |

TECHNICAL FIELDS
SEARCHED      (IPC)

The present search report has been drawn up for all claims

| Place of search | Date of completion of the search | Examiner |
|---|---|---|
| Berlin | 8 December 2022 | Dydenko, Igor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 4481

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2022

| Patent document cited in search report | | Publication date | Patent family member(s) | | Publication date |
|---|---|---|---|---|---|
| WO 0213687 | A1 | 21-02-2002 | AU | 8103401 A | 25-02-2002 |
| | | | US | 6485417 B1 | 26-11-2002 |
| | | | WO | 0213687 A1 | 21-02-2002 |
| US 2003233032 | A1 | 18-12-2003 | AT | 404118 T | 15-08-2008 |
| | | | AU | 2003217652 A1 | 09-09-2003 |
| | | | EP | 1485023 A2 | 15-12-2004 |
| | | | US | 2003233032 A1 | 18-12-2003 |
| | | | WO | 03073211 A2 | 04-09-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KARUNANAYAKA, P.R. ; LEE, E.-Y. ; LEWIS, M.M. ; SEN, S. ; ESLINGER, P.J. ; YANG, Q.X. ; HUANG, X.** Default mode network differences between rigidity- and tremor-predominant Parkinson's disease. *Cortex,* 2017, vol. 81, 239-250